(19) Europäisches Patentamt
European Patent Office
Office européen des brevets

(11) Veröffentlichungsnummer : **0 151 725**
**B1**

(12) # EUROPÄISCHE PATENTSCHRIFT

(45) Veröffentlichungstag der Patentschrift : **02.03.88**

(51) Int. Cl.[4] : **C 07 C 87/62**, C 07 C 85/26, C 07 C 87/60

(21) Anmeldenummer : **84114821.6**

(22) Anmeldetag : **06.12.84**

(54) **Verfahren zur Herstellung von nitrosaminfreien N,N-disubstituierten nitroaromatischen Aminen und deren Stabilisierung gegen Nitrosaminbildung.**

(30) Priorität : **14.12.83 DE 3345157**

(43) Veröffentlichungstag der Anmeldung : **21.08.85 Patentblatt 85/34**

(45) Bekanntmachung des Hinweises auf die Patenterteilung : **02.03.88 Patentblatt 88/09**

(84) Benannte Vertragsstaaten : **AT BE CH DE FR GB IT LI NL**

(56) Entgegenhaltungen :
**DE-A- 2 831 119**
**US-A- 4 226 789**
**JOURNAL OF ORGANIC CHEMISTRY, Band 44, Nr. 5, 2. März 1979, Seiten 784-786, Columbus, Ohio, US; R.F. EIZEMBER et al.: "Destruction of nitrosamines. Treatment of nitrosamines with various acids and halogens"**

(73) Patentinhaber : **HOECHST AKTIENGESELLSCHAFT**
**Postfach 80 03 20**
**D-6230 Frankfurt am Main 80 (DE)**

(72) Erfinder : **Heinrich, Rudolf, Dr.**
**Taunusstrasse 19**
**D-6233 Kelkheim (Taunus) (DE)**
Erfinder : **Albrecht, Konrad, Dr.**
**Sodener Strasse 64**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 151 725 B1

Jouve, 18, rue St-Denis, 75001 Paris, France

**Beschreibung**

Unter N,N-disubstituierten nitroaromatischen Aminen werden im folgenden N,N-disubstituierte Aminonitroaromaten, die am aromatischen Kern gegebenenfalls zusätzlich substituiert sind, verstanden, welche durch Nitrierung entsprechender aromatischer Ausgangsverbindungen mit konzentrierter Salpetersäure oder mit Nitriersäure (konzentrierte Salpetersäure/Schwefelsäure) und übliche Aufarbeitung sowie weitere Umsetzung der erhaltenen aromatischen Nitroverbindungen zu den N,N-disubstituierten Aminonitroaromaten erhalten wurden.

N,N-disubstituierte Aminonitroaromaten sind chemisch und technisch wichtige Verbindungen für verschiedene Einsatzgebiete, wie z. B. den Arzneimittel- und insbesondere den Pflanzenschutzmittelsektor.

Von besonderer wirtschaftlicher Bedeutung sind z. B. die bekannten herbiziden Produkte Trifluralin (2,6-Dinitro-4-trifluormethyl-N,N-dipropylanilin), Isopropalin (2,6-Dinitro-4-isopropyl-N,N-dipropylanilin) sowie auch Benfluralin, Profluralin, Nitralin, Oryzalin.

Die Herstellung z. B. des Trifluralins erfolgt in der Weise, daß 4-Chlorbenzotrifluorid in Gegenwart von Schwefelsäure mit Salpetersäure zum 3,5-Dinitro-4-chlorbenzotrifluorid nitriert wird, aus welchem man dann durch Aminierung mit dem sekundären Di-n-propylamin das 2,6-Dinitro-4-trifluormethyl-N,N-dipropylanilin erhält. Diese Synthese läßt sich durch folgendes Reaktionsschema wiedergeben :

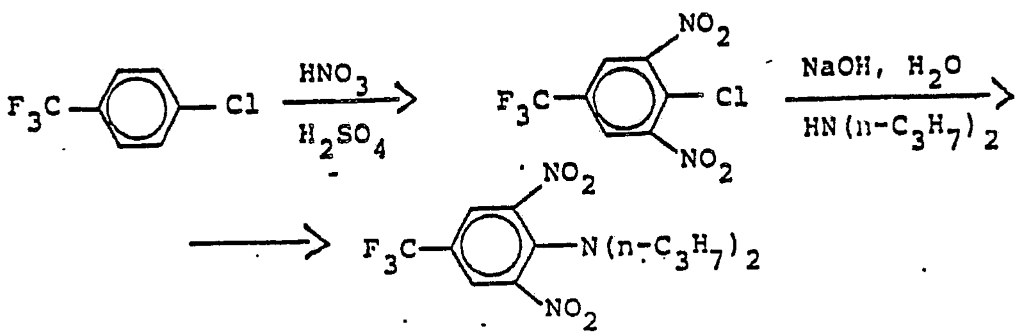


Bei der Herstellung, Lagerung und Verarbeitung solcher N,N-disubstituierten Aminonitroaromaten kann es bekanntlich, besonders bei erhöhten Temperaturen, zur Bildung geringer Mengen von Dialkylnitrosaminen kommen, die u.a. aus überschüssigem sekundären Amin entstehen können. Da verschiedene solcher Nitrosamine im Tierversuch eine cancerogene Wirkung zeigten, stellte sich dem Fachmann in verstärktem Maße das Problem, nitrosaminarme bzw. nitrosaminfreie Produkte herzustellen und außerdem auch unerwünschte nachträgliche Nitrosaminbildugen in den Produkten zu verhindern, wobei der vom Gesetzgeber ggfs. noch tolerierte Nitrosamingehalt bei $\leq$ 1 ppm liegt. Moderne Analysenmethoden ermöglichen es, die genannten Nitrosamine bis in Konzentrationsbereiche von 0,1 ppm (0,1 mg/kg) nachzuweisen.

Im Trifluralin z. B. entsteht Nitrosamin vermutlich durch Nitrosierung des in der letzten Reaktionsstufe eingesetzten Di-n-propylamins durch noch im Produkt vorhandene oder bei dessen thermischer Belastung sich bildende Nitrosierungs-mittel, wodurch der Nitrosamingehalt in dem Produkt z. B. bis auf hundert oder mehr ppm ansteigen kann.

Die zur Entfernung von Nitrosaminen bisher entwickelten Verfahren beruhen im allgemeinen darauf, daß man die von der Nitrierungsreaktion im Reaktionsprodukt noch vorhandenen Nitrosierungsmittel entfernt, um dadurch Nitrosierungsreaktionen zu verhindern, oder daß man bereits entstandene Nitrosaminmengen durch Halogenwasserstoff oder durch Halogene bzw. durch Halogen abspaltende Verbindungen zersetzt, das Produkt anschließend mit Basen behandelt und erneut aufarbeitet.

Nach dem Verfahren der DE-A 29 20 448 (EP-A 19281 wird zur Entfernung der von der Nitrierungsreaktion noch vorhandenen Nitrosierungskomponenten das Reaktionsgemisch ca. 1 Stunde bei 95 - 100° C mit Wasserdampf behandelt und anschließend mit dem Di-n-propylamin umgesetzt.

Ähnlich wird nach dem in der DE-A 28 40 551 beschriebenen Verfahren gearbeitet, in der Absicht, es gar nicht erst zur Bildung von Nitrosaminenkommen zu lassen. Dazu wird das nitrierte Vorprodukt in Gegenwart von $Na_2CO_3$ oder NaOH in wäßriger Phase bei 50 - 100° C durch ca. 1-stündiges Durchleiten von $N_2$, $CO_2$ oder Luft von noch vorhandenem Nitrosierungsmittel befreit und anschließend mit Di-n-propylamin umgesetzt. Die DE-OS 2926947 beschreibt ebenfalls ein Verfahren zur Reinigung des nitrierten Vorproduktes von Nitrosierungskomponenten durch Abkühlung des Nitrieransatzes auf eine Temperatur unterhalb des Erstarrungspunktes und anschließendem Abtrennen des gewünschten reinen, auskristallisierten Vorproduktes.

Obwohl die 3 vorstehend beschriebenen Verfahren der weitgehenden Entfernung der die Nitrosierung des Amins bewirkenden Bestandteile dienen, führen sie lediglich zu Produkten mit einem

verringerten Nitrosamingehalt. Außerdem verhindern diese Methoden nicht die Nitrosaminbildung bei längerer thermischer Behandlung der Dinitroaniline. So haben Untersuchungen ergeben, daß in Trifluralin, welches unter Anwendung der oben beschriebenen Reinigungsverfahren hergestellt wurde, nach einer thermischen Behandlung von 12 - 15 Stunden bei 70 - 80° C der Di-n-propylnitrosamin-Wert von 0,6 ppm auf 2,5 ppm und darüber anstieg.

In der DE-A 28 31 119 wird ein Verfahren zur Reinigung von bereits fertigem technischem Trifluralin durch Behandlung desselben mit wäßriger oder gasförmiger Salzsäure bei erhöhten Temperaturen beschrieben. Durch diesen ca. 2-4 Stunden währenden Prozeß wird das vorhandene Nitrosamin zerstört. Die Salzsäure wird anschließend mit $NaHCO_3$ neutralisiert und das gereinigte technische Produkt erneut aufgearbeitet.

In der DE-A 28 35 530 werden die Halogene Chlor, Brom sowie deren Derivate Bromchlorid, N-Bromsuccinimid, N-Chlorsuccinimid u.a. für die Zersetzung der Nitrosamine eingesetzt. Auch hierbei ist eine anschließende Neutralisation mit Alkali erforderlich, ebenso nach dem in der DE-A 28 37 529 beschriebenen Verfahren zur Zersetzung der Nitrosamine mit Phosphorhalogeniden, Schwefelhalogeniden oder $TiCl_4$.

Die 3 zuletzt beschriebenen Prozesse führen zu nitrosaminarmen Produkten, setzen allerdings eine längere thermische Behandlung des technischen Produktes mit anschließender Neutralisation der gereinigten Ware mit Alkali und einen sich daran anschließenden Aufarbeitungsschritt voraus. Außerdem können sie in den meisten Fällen einen Wiederanstieg des Nitrosamin-Wertes bei längerer Thermischer Behandlung des Produktes nicht verhindern. Sie führen daher zwar zu einer Senkung des Nitrosamingehaltes auf ein niedriges Niveau, nicht aber zu einer Stabilisierung gegen erneute Nitrosaminbildung.

Alle bisher bekannt gewordenen Verfahren haben somit u.a. den Nachteil, daß sie einen zusätzlichen Aufarbeitungsschritt erfordern und/oder keine ausreichende Stabilisierung technischer, N,N-disubstituierter Aminonitroaromaten bei längerer Lagerung unter thermischer Einwirkung bzw. Behandlung garantieren.

Es bestand somit die Aufgabe, ein Verfahren zu entwickeln, das die geschilderten Nachteile überwindet und eine Eliminierung sowie eine Verhinderung der Neubildung von Nitrosaminen in N,N-disubstituierten Aminonitroaromaten ermöglicht. Es wurden daher Verbindungen verschiedener chemischer Strukturen auf eine mögliche Nitrosamin abbauende sowie eine erneute Nitrosaminbildung verhindernde Wirkung untersucht. Dabei zeigte sich überraschenderweise, daß Sulfonsäuren in N,N-disubstituierten Aminonitroaromaten einen stark Nitrosamin abbauenden und einen möglichen Nitrosamin-Wiederanstieg verhindernden Effekt besitzen. Dieser Effekt konnte z. B. mit aliphatischen, aromatischen und araliphatischen Sulfonsäuren, aber auch mit Chlor- und Amidosulfonsäure erzielt bzw. nachgewiesen werden. Dabei waren sowohl in der Schmelze der zu behandelnden N,N-disubstituierten Aminonitroaromaten lösliche, z. B. Dodecylbenzolsulfonsäure, als auch unlösliche Verbindungen, wie z. B. p-Toluolsulfonsäure, wirksam. Die für einen vollständigen Abbau der Nitrosamine erforderlichen Reaktionszeiten liegen, abhängig von der Reaktionstemperatur, zwischen 1 und 10 Stunden, wobei die Reaktionstemperaturen zwischen 20 und 120 °C liegen können. Für einen vollständigen Abbau in üblichen technischen Produkten mit anschließender Stabilisierung waren, je nach Nitrosamingehalt, Löslichkeit in der Schmelze und sonstigen Reaktionsbedingungen, Sulfonsäuremengen bis zu etwa 1 Gew.-%, vorzugsweise 0,05 bis 0,5 Gew.-%, erforderlich, bezogen auf die eingesetzte N,N-disubstituierte aminonitroaromatische Verbindung.

Es können aber unbeschadet auch größere Mengen als 1 Gew.-% Sulfonsäure zugesetzt werden, falls dies in manchen Fällen u.a. aus anderen Gründen wünschenswert bzw. vorteilhaft erscheint.

Das Verfahren wird zweckmäßigerweise so durchgeführt, daß der geschmolzenen, Nitrosaminhaltigen N-N-disubstituierten aminonitroaromatischen Verbindung die erforderliche Menge Sulfonsäure bei einer Temperatur z. B. zwischen 50 und 90 °C zugesetzt wird und anschließend bei dieser Temperatur noch einige Stunden nachgerührt wird. Bei den in der Schmelze löslichen Verbindungen, wie z. B. Dodecylbenzolsulfonsäure, kann auf das Nachrühren verzichtet werden. Aufarbeitungsvorgänge sind nicht mehr erforderlich, da die benötigten geringen Mengen an Sulfonsäure-Stabilisator die Weitere Verwendung des N,N-disubstituierten aminonitroaromatischen Produktes im allgemeinen, z. B. bei dessen Formulierung als Pflanzenschutzmittel, nicht stören. Die in der Schmelze unlöslichen Sulfonsäurestabilisatoren können jedoch, z. B. bei einem dem ersten Formulierungsschritt sich anschließenden Reinigunsprozeß, auch abfiltriert werden. In besonderen Fällen kann auch eine Neutralisation mit basischen Verbindungen kurz vor der Weiterverwendung des N,N-disubstituierten aminonitroaromatischen Produktes nötig bzw. vorteilhaft sein.

Im Gegensatz zu den überraschenden Ergebnissen der vorliegenden Erfindung ist z. B. in J. Org. Chem. 44, (5) (1979), S. 784 beschrieben, daß Säuren, wie z. B. p-Toluolsulfonsäure, zur Entfernung von Nitrosaminen aus Dinitroanilinen nur sehr wenig oder gar nicht wirksam sind, während mit Schwefelsäure Nitrosamine nicht abgebaut sondern lediglich extrahiert werden können.

Gegenstand der Erfindung ist daher ein Verfahren zur Herstellung von nitrosaminfreien N,N-disubstituierten nitroaromatischen Aminen durch Abbau ihrer herstellungsbedingten Nitrosaminbestandteile und Stabilisierung der Produkte gegen erneute Nitrosaminbildung, dadurch gekennzeichnet, daß man die Amine in flüssiger bzw. geschmolzener Form mit einer Sulfonsäure behandelt. Unter N,N-disubstituierten nitroaromatischen Aminen sind erfindungsgemäß die eingangs erläuterten und definier-

ten Verbindungen bzw. Produkte zu verstehen, wobei die pestiziden Wirkstoffe bevorzugt sind.

Als Substituenten am Aminstickstoff der N,N-disubstituierten nitroaromatischen Amine, die gleich oder verschieden sein oder zusammen mit dem Aminstickstoff einen heterocyclischen Ring bilden können, sind beispielsweise zu nennen :

Alkyl-, Alkenyl-, Alkinylgruppen mit bis zu 12 C-Atomen, vorzugsweise mit bis zu 5 C-Atomen, die ggfs. substituiert, vorzugsweise durch Halogen, insbesondere Cl oder Br, OH, Hydroxy-($C_1$-$C_4$)-alkyl oder Cyclo-($C_3$-$C_6$)-alkyl substituierte sind, ferner Aryl- oder Aralkylgruppen, insbesondere Phenyl oder Benzyl, die ggfs. substituiert, vorzugsweise durch Halogen, $CF_3$ ($C_1$-$C_4$)-Alkyl oder ggfs. substituiertes Phenoxy substituiert sind.

Die aromatischen Kerne der N,N-disubstituierten nitroaromatischen Amine, die bevorzugt Benzolringe darstellen, tragen im allgemeinen z. B. eins bis drei, insbesondere eins oder zwei, vorzugsweise zwei, Nitrogruppen und gegebenenfalls einen oder mehrere weitere Substituenten, insbesondere z. B. Halogen, vorzugsweise Cl oder Br, $CF_3$, ($C_1$-$C_{12}$)-alkyl, Halogen-($C_1$-$C_{12}$)-Alkyl, ($C_1$-$C_4$)-Alkoxy, Halogen-($C_1$-$C_4$)-alkoxy, ggfs. substituiertes Phenoxy, ($C_2$-$C_5$)-Alkenyl, Halogen-($C_2$-$C_5$)-alkenyl, ($C_2$-$C_5$)-Alkinyl, Halogen-($C_2$-$C_5$)-alkinyl, $-SO_2-$($C_1$-$C_4$)-alkyl, $-SO_2-NH_2$, $-CN$.

Besonders bevorzugt sind N,N-disubstituierte Dinitroanilinoverbindungen.

Als Sulfonsäuren kommen in Frage :

Aliphatische, aromatische oder araliphatische Sulfonsäuren, vozugsweise mit bis zu 18 C-Atomen, Chlorsulfonsäure, Amidosulfonsäure oder Gemische aus diesen Sulfonsäuren.

Die Zusatzmengen an Sulfonsäuren sind nicht kristisch. Sie richten sich im allgemeinen nach dem Gehalt an Nitrosaminverunreinigungen bzw. an potentiellen Nitrosaminbildnern in dem zu reinigenden und zu stabilisierenden N,N-disubstituierten nitroaromatischen Amin.

Bei üblichen technischen Produkten betragen die erforderlichen Zusatzmengen an Sulfonsäuren im allgemeinen bis zu 1 Gew.-%, vorzugsweise 0,05 bis 0,5 Gew.-%, bezogen auf das zu behandelnde N,N-disubstituierte nitroaromatische Amin, wobei das letztere in geschmolzener bzw. in flüssiger Form, ggfs. unter Zusartz von inerten organischen Lösungs- oder Verdünnungsmitteln, mit der Sulfonsäure vermischt und das Gemisch anschließend noch einige Zeit, vorzugsweise 1 bis 10 Stunden, erwärmt wird, vorzugsweise auf Temperaturen bis zu 120 °C, insbesondere auf 50 bis 90 °C, ggfs. unter Rühren.

Eine Stabilisierung der genannten technischen Produkte gegen erneute Nitrosaminbildungen ist insbesondere bei thermischer Relastung z. B. während des Aufschmelzvorganges, oder bei längerer Lagerung, insbesondere bei erhöhten Temperaturen, erforderlich.

Erfindungsgemäß können die einzusetzenden Sulfonsäuren bzw. ihre Umsetzungsprodukte in dem von Nitrosaminen befreiten technischen Produkt verbleiben, sie können aber auch kurz vor dessen Weiterverwendung neutralisiert oder während der Weiterverwendung beim ersten Reinigungsschritt abfiltriert werden.

Als N,N-disubstituierte Dinitroanilinoverbindungen kommen u.a. insbesondere die wichtigen herbiziden Verbindungen Trifluralin und Isopropalin in Frage. Zu den erfindungsgemäß verwendbaren Sulfonsäuren gehören insbesondere aliphatische Sulfonsäuren, wie z. B. ($C_2$-$C_{10}$)-Alkansulfonsäuren, Toluolsulfonsäure (o-, m-, p-), 1,3-Xylol-4-sulfonsäure, 1,4-Xylol-2-sulfonsäure, Dodecylbenzolsulfonsäure, Isododecylbenzolsulfonsäure, 1-Naphthol-4-sulfonsäure, p-Aminobenzolsulfonsäure, Chlorsulfonsäure, Amidosulfonsäure.

Die Bestimmung der Nitrosaminmengen erfolgte durch eine gaschromatographische Analysenmethode mit einer Empfindlichkeit bis etwa $\leq$ 0,1 ppm (0,1 mg/kg) (vgl. DE- A 28 31 119).

Die Erfindung wird durch folgende Beispiele näher erläutert.

## Beispiel 1

Es wurden 50 g Trifluralin mit einem Di-n-propylnitrosamin-Gehalt von 58 ppm in einem Rührkolben auf 80 °C erwärmt, danach 0,1 g p-Toluolsulfonsäure zugesetzt und 5 Stunden bei der angegebenen Temperatur gerührt. Danach wurde das Produkt ohne Rühren erkalten gelassen, wobei sich p-Toluolsulfonsäure am Boden des Reaktionsgefäßes absetzte. Das so gereinigte Trifluralin wurde mit Hilfe der bereits erwähnten gaschromatographischen Methode auf den Di-n-propyl-nitrosamin-Gehalt untersucht. Die Analyse ergab einen Nitrosamingehalt von $<$ 0,2 ppm.

## Beispiel 2

Es wurden 50 g Trifluralin mit einem Di-n-propylnitrosamin-Gehalt von 58 ppm auf 80 °C erwärmt und danach 0,3 g Dodecylbenzolsulfonsäure zugegeben. Das Gemisch wurde ca. 5 Minuten nachgerührt und anschließend noch 2 Stunden bei der angegebenen Temperatur gehalten. Danach wurde erkalten lassen und eine Probe zur analytischen Bestimmung des Nitrosamin-Gehaltes entnommen. Die Analyse ergab einen Di-n-propylnitrosamingehalt von $\leq$ 0,1 ppm.

## Beispiel 3

Es wurden 50 g Trifluralin mit einem Di-n-propylnitrosamin-Gehalt von 30 ppm bei 80 °C mit 0,5 g

Butansulfonsäure nach der im Beispiel 1 angegebenen Methode behandelt. Nach 6 Stunden wurde eine Probe zur analystischen Bestimmung des Nitrosamin-Gehaltes entnommen. Die Analyse ergab einen Di-n-propylnitrosamingehalt von < 0,3 ppm.

Beispiel 4

Es wurden 50 g Trifluralin mit einem Di-n-propylnitrosamin-Gehalt von 30 ppm bei 90 °C mit 0,3 g Amidosulfonsäure nach der im Beispiel 1 angegebenen Methode behandelt. Nach 8 Stunden wurde eine Probe zur analystischen Bestimmung des Nitrosamin-Gehaltes entnommen. Die Analyse ergab einen Di-n-propylnitrosamingehalt von < 0,3 ppm.

Beispiel 5

Es wurden 50 g Trifluralin mit einem Di-n-propylnitrosamin-Gehalt von 58 ppm bei 70 °C mit 0,2 g Chlorsulfonsäure nach der im Beispiel 1 angegebenen Methode behandelt. Nach 5 Stunden wurde eine Probe entnommen, deren Analyse einen Di-n-propylnitrosamingehalt von < 0,1 ppm ergab.

Beispiel 6

Es wurden 50 g Trifluralin mit einem Di-n-propylnitrosamin-Gehalt von 30 ppm bei 90 °C mit 0,3 g Octylbenzolfonsäure nach der im Beispiel 1 angegebenen Methode behandelt. Nach 6 Stunden wurde eine Probe zur analystischen Bestimmung entnommen. Die Analyse ergab einen Di-n-propylnitrosamingehalt von < 0,2 ppm.

Beispiel 7

Es wurden 50 g Isopropalin mit einem Di-n-propylnitrosamin-Gehalt von 15 ppm bei 70 °C mit 0,2 g p-Toluosulfonsäure analog Beispiel 1 behandelt. Nach 3 Stunden Reaktionszeit betrug der Gehalt an Di-n-propylnitrosamin < 0,2 ppm.

Beispiel 8

Es wurden 50 g Trifluralin mit einem Di-n-propylnitrosamin-Gehalt von 30 ppm bei 70 °C mit 0,2 g p-Toluolsulfonsäure nach der im Beispiel 1 angegebenen Methode behandelt. Nach einer Reaktionszeit von 5 Stunden wurde das Produkt mit 50 g dest. Wasser versetzt, 30 Minuten bei 50 °C gerührt und mit Natriumcarbonat auf pH 8 gebracht. Anschließend wurde bei der angegebenen Temperatur noch ca. 30 Minuten weitergerührt und auf pH 8 gehalten. Danach wurden die wäßrige Phase und das behandelte Trifluralin auf ihren Nitrosamin-Gehalt untersucht. Ergebnis : < 0,1 ppm Di-n-propylnitrosamin in der wäßrigen Phase : < 0,2 ppm Di-n-propylnitrosamin im behandelten Trifluralin

Beispiel 9

Nitrosamin-Abbau und Stabiliserung gegen einen Wiederanstieg des Nitrosamingehaltes durch erfindungsgemäße Zusätze

Es wurden 100 g Trifluralin mit einem Di-n-propylnitrosamin-Gehalt von 0,6 ppm geschmolzen und die Ansatzmenge halbiert. Der einen Hälfte wurden 0,3 Gew.-% Dodecylbenzolsulfonsäure zugesetzt und danach beide Proben 1 Woche bei 70 °C gelagert. Die-n-propylnitrosamin-Bestimmung ergab bei der mit Dodecylbenzolsufonsäure versetzten Probe einen Gehalt von < 0,2 ppm Di-n-propylnitrosamin, während der Gehalt in der nicht stabilisierten Probe auf 2,5 ppm Die-n-propylnitrosamin angestiegen war.

**Patentansprüche**

1. Verfahren zur Herstellung von nitrosaminfreien N,N-disubstituierten nitroaromatischen Aminen durch, Abbau ihrer herstellungsbedingten Nitrosaminbestandteile und Stabilisierung der Produkte gegen erneute Nitrosaminbildung, dadurch gekennzeichnet, daß man die Amine in flüssiger bzw. geschmolzener Form mit einer Sulfonsäure behandelt.

2. Verfahren nach Anspruch 1, gekennzeichnet, daß man die Amine mit aliphatischen, aromatischen oder araliphatischen Sulfonsäuren, Chlorsulfonsäure, Amidosulfonsäure oder deren Gemischen behandelt.

3. Verfahren nach Anspruch 2, dadurch gekennzeichnet, daß man die Amine mit Dodecylbenzolsulfonsäure, Butansulfonsäure oder Chlorsulfonsäure behandelt.

4. Verfahren nach Ansprüchen 1 bis 3, dadurch gekennzeichnet, daß man als Amin Trifluralin oder Isopropalin einsetzt.

5. Verfahren nach Ansprüchen 1 bis 4, dadurch gekennzeichnet, daß man das Amin mit bis zu 1 Gew.-%, vorzugsweise 0,05 bis 0,5 Gew.-%, Sulfonsäure, bezogen auf das Amin, behandelt.

6. Verfahren nach Ansprüchen 1 bis 5, dadurch gekennzeichnet, daß der Suflonsäurezusatz bei Temperaturen zwischen 20 und 120 °C erfolgt und das Amin anschließend 1 bis 10 Stunden auf eine erhöhte Temperatur, vozugsweise auf 50 bis 90 °C, erwärmt wird.

7. Verfahren nach Ansprüchen 1 bis 6, dadurch gekennzeichnet, daß dem zu reinigenden Amin ein inertes organisches Lösungemittel oder Verdünnungsmittel zugesetzt wird.

8. Verfahren zur Stabilisierung von N,N-disubstituierten Dinitroanilin-Verbindungen gegen die Bildung von Nitrosaminen, dadurch gekennzeichnet, daß man den Aminen bis zu 1 Gew.-% an Dodecylbenzolsulfonsäure zufügt.

9. Mittel, die N,N-disubstituierte Dinitroanilin Verbindungen gegen die Bildung von Nitrosaminen stabilisieren, dadurch gekennzeichnet, daß sie neben der Anilinverbindung bis zu 1 Gew.-% einer Sulfonsäure enthalten.

10. Mittel gemäß Anspruch 9, dadurch gekennzeichnet, daß sie eine aromatische Sulfonsäure mit bis zu 18 C-Atomen enthalten.

11. Mittel gemäß Anspruch 9, dadurch gekennzeichnet, daß sie Dodecylbenzolsulfonsäure enthalten.

**Claims**

1. A process for the preparation of nitrosaminefree N,N-disubstituted nitroaromatic amines by degradation of their nitrosamine components formed during their preparation, and by stabilization of the products against renewed formation of nitrosamines, wherein the amines are treated in liquid or molten form with a sulfonic acid.

2. The process as claimed in claim 1, wherein the amines are treated with aliphatic, aromatic or araliphatic sulfonic acids, chlorosulfonic acid, amidosulfonic acid, or mixtures thereof.

3. The process as claimed in claim 2, wherein the amines are treated with dodecylbenzenesulfonic acid, butanesulfonic acid or chlorosulfonic acid.

4. The process as claimed in claim 1, wherein trifluralin or isopropalin is used as the amine.

5. The process as claimed in claim 1, wherein the amine is treated with up to 1 % by weight, preferably 0.05 to 0.5 % by weight, based on the amine, of sulfonic acid.

6. The process as claimed in claim 1, wherein the addition of the sulfonic acid takes place at temperatures between 20 and 120 °C, and wherein the amine is then heated for 1 to 10 hours at an elevated temperature, preferably at 50 to 90 °C.

7. The process as claimed in claim 1, wherein an inert organic solvent or diluent is added to the amine which is to be purified.

8. A process for stabilizing N,N-disubstituted dinitroaniline compounds against the formation of nitrosamines characterized in that dodecylbenzenesulfonic acid is added to the amines in an amount of up to 1 % b.w.

9. Compositions which stabilize N,N-disubstituted dinitroaniline compounds against the formation of nitrosamines characterized in that they contain up to 1 % b.w of a sulfonic acid besides the aniline compound.

10. Composition as claimed in claim 9, characterized in that they contain an aromatic sulfonic acid having up to 18 C-atoms.

11. Compositions as claimed in claim 9, characterized in that they contain dodecylbenzenesulfonic acid.

**Revendications**

1. Procédé pour la préparation d'amines nitroaromatiques N,N-disubstituées exempes de nitrosamines, par dégradation de leurs constituants nitrosamines dus aux conditions de préparation, et stabilisation des produits vis-à-vis d'une reformation des nitrosamines, caractérisé en ce qu'on traite les amines sous forme liquide ou fondue avec un acide sulfonique.

2. Procédé selon la revendication 1, caractérisé en ce qu'on traite les amines avec des acides sulfoniques aliphatiques, aromatiques ou araliphatiques, l'acide chlorosulfonique, l'acide amidosulfonique ou leurs mélanges.

3. Procédé selon la revendication 2, caractérisé en ce qu'on traite les amines avec l'acide dodécylbenzènesulfonique, l'acide butanesulfonique ou l'acide chlorosulfonique.

4. Procédé selon les revendications 1 à 3, caractérisé en ce qu'on utilise comme amine la trifluraline ou l'isopropaline.

5. Procédé selon les revendications 1 à 4, caractérisé en ce qu'on traite l'amine avec jusqu'à 1 % en poids, de préférence 0,05 à 0,5 % en poids d'acide sulfonique, par rapport à l'amine.

6. Procédé selon les revendications 1 à 5, caractérisé en ce que l'adjonction d'acide sulfonique s'effectue à des températures comprises entre 20 et 120 °C, et que l'amine est ensuite chauffée pendant 1

à 10 heures à une température élevée, de préférence de 50 à 90°C.

7. Procédé selon les revendications 1 à 6, caractérisé en ce que l'on ajoute à l'amine à purifier un solvant ou un diluant organique inerte.

8. Procédé pour stabiliser des composés dinitroanilines N,N-disubstitués vis-à-vis de la formation de nitrosamines, caractérisé en ce qu'on ajoute aux amines jusqu'à 1 % en poids d'acide dodécylbenzènesulfonique.

9. Agents qui stabilisent les composés dinitroanilines N,N-disubstitués vis-à-vis de la formation de nitrosamines, caractérisés en ce qu'ils contiennent, outre le composé de l'aniline, jusqu'à 1 % en poids d'un acide sulfonique.

10. Agents selon la revendication 9, caractérisés en ce qu'ils contiennent un acide sulfonique aromatique ayant jusqu'à 18 atomes de carbone.

11. Agents selon la revendication 9, caractérisés en ce qu'ils contiennent de l'acide dodécylbenzènesulfonique.